# EUROPÄISCHE PATENTSCHRIFT

(11) **EP 1 838 368 B1**
(45) Veröffentlichungstag und Bekanntmachung des Hinweises auf die Patenterteilung: **16.01.2019**
(21) Anmeldenummer: 05812740.8
(22) Anmeldetag: 12.12.2005
(51) Int. Cl.: A61M 5/315

(54) **INJEKTIONSVORRICHTUNG MIT EINER DOSIERVORRICHTUNG**
INJECTION DEVICE WITH A DOSING MECHANISM
DISPOSITIF D'INJECTION AVEC UN ÉLÉMENT DE DOSAGE

(30) Priorität: 10.01.2005 DE 102005001159
(43) Veröffentlichungstag der Anmeldung: 03.10.2007
(73) Patentinhaber: TecPharma Licensing AG, 3401 Burgdorf (CH)
(72) Erfinder: BURREN, Stefan, CH-3047 Bremgarten (CH); MOSER, Ulrich, CH-3412 Heimiswil (CH); SCHOCH, Roland, CH-4574 Nennigkofen (CH); SCHRUL, Christian, CH-3427 Utzenstorf (CH); ZURFLUEH, Philippe, CH-4133 Pratteln (CH)
(86) Internationale Anmeldenummer: PCT/CH2005/000740
(87) Internationale Veröffentlichungsnummer: WO 2006/072188

(56) Entgegenhaltungen:
- EP-A- 0 937 471
- WO-A-01/95959
- WO-A-99/38554
- WO-A-2004/007001
- WO-A-2004/030730
- WO-A-2004/078239
- WO-A-2005/018721
- WO-A-2005/046770
- US-A- 5 271 527

## Beschreibung

Die vorliegende Erfindung bezieht sich auf eine Injektionsvorrichtung mit einer Dosiervorrichtung zur Einstellung einer aus der Injektionsvorrichtung abzugebenden Dosis, insbesondere auf eine Injektionsvorrichtung mit einer Dosiervorrichtung zur Einstellung einer oder mehrerer fest vorgegebener Dosiseinheiten oder zur Vorbereitung der Injektionsvorrichtung zur Abgabe einer oder mehrerer fest voreingestellter Dosismengen z. B. aus einer in die Injektionsvorrichtung eingelegten Ampulle.

Eine Vorrichtung, wie die Erfindung sie betrifft, ist beispielsweise aus der WO 97/36626 bekannt. Die Vorrichtung weist ein Gehäuse mit einem Reservoir für das Produkt auf. In dem Reservoir ist ein Kolben aufgenommen, der bei einer Verschiebung in eine Vorschubrichtung das Produkt aus dem Reservoir durch einen Auslass des Reservoirs verdrängt. Eine Zahnstange wirkt als Kolbenstange und schiebt den Kolben in Vorschubrichtung. Im Gehäuse ist ferner ein Antriebsglied relativ zum Gehäuse in und gegen die Vorschubrichtung verschiebbar aufgenommen, das bei einer Verschiebung in Vorschubrichtung die Zahnstange mitnimmt. Hierfür greift das Antriebsglied mit Mitnehmern in Zahnreihen der Zahnstange ein. Zum Einstellen derjenigen Produktmenge, die mit einem Hub verabreicht wird, d.h. durch die Betätigung einer Dosiereinrichtung, wird das Antriebsglied in einer vorderen Stellung um eine eingestellte Dosisweglänge manuell gegen die Vorschubrichtung zurückgezogen. Dabei gleiten die Mitnehmer des Antriebsgliedes über die Zähne der Zahnreihen der Zahnstange und geben dabei elastisch nach. Ein Zurückverschieben der Zahnstange wird durch relativ zum Gehäuse verschiebegesichert aufgenommene Sperrmittel verhindert. Die Sperrmittel wirken mit einer der Zahnreihen der Zahnstange derart zusammen, dass die Sperrmittel eine Verschiebung der Zahnstange gegen die Vorschubrichtung verhindern: Durch elastisches Nachgeben erlauben sie eine Verschiebung der Zahnstange in Vorschubrichtung. Durch die Betätigung des Antriebsknopfes wird mittels des Antriebsglieds die eingestellte Dosisweglänge von der Zahnstange, bzw. dem Kolben, zurückgelegt, sodass die eingestellte Dosis durch den Auslass des Reservoirs ausgeschüttet wird.

Die WO2004078239 beschreibt einen Antriebsmechanismus für ein Injektionsgerät wo eine Einstellbewegung des Einstellelementes auf eine Antriebshülse übertragen wird, wobei eine mit der Antriebshülse im Eingriff stehende Kolbenstange nicht bewegt wird. Bei der Dosisausschüttung wird eine axiale Bewegung der Antriebshülse in eine Schraubbewegung der Kolbenstange umgesetzt. Die WO2004078239 offenbart die Merkmale des einleitenden Teils des Anspruchs 1.

Des Weiteren beschreiben die EP0937471, US5271527 und WO2004030730 Injektionsvorrichtungen für die Verabreichung eines Medikaments sowie diese dem Fachmann aus dem Stand der Technik bekannt sind.

Es ist eine Aufgabe der vorliegenden Erfindung eine Injektionsvorrichtung mit einer Dosiereinrichtung vorzuschlagen, welche das Einstellen einer Dosis und insbesondere das exakte Einstellen und Ausschütten einer kleinen Dosismenge vereinfacht.

Diese Aufgabe wird durch eine Dosiervorrichtung gemäß Anspruch 1 gelöst. Vorteilhafte Ausführungsformen ergeben sich aus den abhängigen Ansprüchen.

Eine erfindungsgemäße Injektionsvorrichtung mit einer Dosiervorrichtung weist ein Einstellelement auf, welches mit einer bevorzugt etwa zylinderförmigen Drehhülse drehbar verbunden ist. Die Drehhülse weist ein Innengewinde und ein Außengewinde mit bevorzugt gleichem Drehsinn auf, welche bevorzugt koaxial zueinander angeordnet sind, so dass die Drehhülse in mindestens einem Bereich ein Innengewinde und ein Außengewinde aufweist, welche sich bevorzugt überlappen und z. B. auch etwa über die gesamte Länge der Drehhülse an der Innenseite und der Außenseite des zylinderförmigen Drehhülsenkörpers angeordnet sein können. Die Gewinde sind bevorzugt als Bewegungsgewinde so ausgebildet, dass die Gewinde nicht selbsthemmend sind und können Gewindegänge oder in Gewindegänge eingreifende Elemente oder Nocken oder auch umlaufende Spiralen sein, welche in die Gewinde jeweils gegenüberliegender Gegengewinde eingreifen oder mit diesen zusammenwirken können. Innerhalb und außerhalb der Drehhülse sind Elemente der Injektionsvorrichtung vorgesehen, welche bevorzugt relativ zueinander verdrehgesichert sind oder welche z.B. bezüglich einem Gehäuse der Injektionsvorrichtung verdrehgesichert sind.

Dabei weist erfindungsgemäß das Innengewinde der Drehhülse eine andere Steigung auf als das Außengewinde der Drehhülse.

Ist z. B. innerhalb der Drehhülse ein Vorschubglied der Injektionsvorrichtung gelagert, welches sich im Gewindeeingriff mit der Drehhülse befindet, so kann eine definierte Bewegung des Vorschubgliedes um eine vorgegebene Distanz in proximale und distale Richtung der Injektionsvorrichtung durch ein Drehen der Drehhülse bewirkt werden, welche in einem Gewindeeingriff mit einem z. B. an der Außenseite der Drehhülse anliegenden Gehäuseteil der Injektionsvorrichtung ist. Hat das Innengewinde der Drehhülse eine kleinere Steigung als das Außengewinde der Drehhülse, so wird, wenn die Drehhülse z. B. um die axiale Länge D aus dem Gehäuse der Injektionsvorrichtung herausgedreht wird, mit welchem die Drehhülse im Gewindeeingriff steht, das Vorschubglied um eine kleinere Distanz d in die gleiche Richtung bewegt, wodurch eine Untersetzung einer Einstellbewegung durch eine kompakte Bauart realisiert werden kann. Somit kann eine Übersetzung eines kleinen funktionalen Weges d in einen größeren Weg D zum einfachen Einstellen z. B. einer Fixdosis realisiert werden.

Ist mit dem Vorschubglied ein Mitnehmer, wie z. B. ein Rastelement verbunden, welches z. B. an einem flexiblen Element oder Arm des Vorschubgliedes vorgesehen ist, so kann ein an dem flexiblen Element vorgesehener z. B. radial nach innen vorstehender Nocken oder Nase über die Zahnung einer als Kolbenstange dienenden Zahnstange oder das Gewinde einer Gewindestange geführt werden, wobei je nach der Größe der axialen Bewegung des Vorschubgliedes relativ zur Zahnstange ein oder mehrere Zähne der Zahnstange durch den elastisch gelagerten Nocken oder Nase überfahren werden, wodurch ein oder mehrere "Klick"-Geräusche erzeugt werden und die aus der Injektionsvorrichtung abzugebende Dosis festgelegt wird. Die so eingestellte Dosis wird durch eine durch ein Zurückschieben oder -drehen der Drehhülse bewirkte Bewegung des Vorschubgliedes oder der Vorschubhülse in distale Richtung, welche durch einen Eingriff der Nocken oder Nasen auf die Zahnstange übertragen wird und an einen unmittelbar oder mittelbar an der Zahnstange anliegenden Stopfen weitergegeben wird, welcher in eine Ampulle zur Verdrängung einer darin enthaltenen Substanz eingeschoben wird, ausgeschüttet.

Bezüglich der prinzipiellen Funktionsweise einer Dosiervorrichtung, welche eine Vorschubhülse oder ein Vorschubglied aufweist, wird auf die deutsche Patentanmeldung mit der Anmeldenummer 10 2004 041 151.4 der Anmelderin verwiesen.

Bei den auf der Innen- und Außenseite der Drehhülse vorgesehenen Gewinde mit unterschiedlicher Steigung ist bevorzugt die Steigung desjenigen Gewindes, welches mit dem Vorschubglied im Eingriff steht, kleiner als die Steigung des gegenüberliegenden Gewindes, welches mit dem Gehäuse der Injektionsvorrichtung im Eingriff steht, bezüglich dessen das Vorschubglied bevorzugt drehsicher gelagert ist, so dass eine bei einem Einstellvorgang durch einen Gewindeeingriff der Drehhülse mit dem Gehäuse erzwungene Drehung der Drehhülse relativ zu dem Gehäuse nicht in eine Drehung des ebenfalls im Gewindeeingriff mit der Drehhülse stehenden Vorschubgliedes, sondern in eine Axialbewegung des Vorschubgliedes relativ zum Gehäuse umgesetzt wird. Dabei kann das Vorschubglied sowohl innerhalb, als auch außerhalb, der Drehhülse mit einem Außen- oder Innengewinde vorgesehen sein. Ebenso ist es möglich, dass die Steigung des Gewindes, mit welchem Drehhülse und Vorschubglied in Eingriff stehen, größer ist als die Steigung des auf der anderen Seite der Drehhülse vorgesehenen Gewindes, wobei in diesem Fall eine kleine Einstellbewegung in eine im Verhältnis dazu größere Axialbewegung des Vorschubgliedes umgesetzt wird.

An der Drehhülse ist ein Einstellelement, wie z. B. ein Bedienknopf vorgesehen.

Die Drehhülse ist drehbar mit dem Einstellelement verbunden, so dass der Bedienknopf z. B. ohne Drehbewegung aus dem Gehäuse der Injektionsvorrichtung herausgezogen werden kann und die drehbar im Bedienknopf gelagerte Drehhülse aus dem Gehäuse der Injektionsvorrichtung herausgedreht wird.

Vorzugsweise sind die an der Drehhülse vorgesehenen Gewinde nicht selbsthemmend, wobei z. B. der Steigungswinkel des Gewindes so gewählt werden kann, dass der Tangens dieses Steigungswinkels größer als der Reibungskoeffizient der aneinander liegenden und im Gewindeeingriff stehenden Materialien ist. Es können auch Schmiermittel, wie z. B. Teflon, verwendet werden, um nicht selbsthemmende Gewindeeingriffe zu realisieren. Ebenso kann z. B. eine Torsionsfeder vorgesehen sein, welche beim Herausziehen oder- drehen der Drehhülse gespannt oder aufgezogen wird und so z. B. mit der Drehhülse verbunden ist, dass beim Einschieben oder -drehen der Drehhülse die Federkraft in Drehrichtung wirkt, so dass auch selbsthemmende Gewinde verwendet werden können, wobei die Hemmung der Gewinde durch die Feder aufgehoben wird.

Bevorzugt sind die Außen- und Innengewinde der Drehhülse koaxial zueinander angeordnet, d. h. die Gewinde überlappen sich in axialer Richtung, wodurch eine kompakte und die Bauhöhe der Injektionsvorrichtung verringernde Bauweise einer Übersetzung bzw. Untersetzung realisiert werden kann.

Vorzugsweise sind Radial- und/oder Axialanschläge vorgesehen, welche z. B. die Bewegung des Vorschubgliedes und/oder der Drehhülse in radialer und/oder axialer Richtung z. B. relativ zum Gehäuse der Injektionsvorrichtung begrenzen können. Beispielsweise können auf dem Vorschubglied zwei in axialer Richtung voneinander beabstandete Anschläge vorgesehen sein, welche mit einem Anschlagselement des Gehäuses so zusammenwirken, dass das Vorschubglied in axialer Richtung der Injektionsvorrichtung relativ zum Gehäuse nur um eine vorgegebene Distanz d bewegt werden kann, wodurch z. B. eine der Distanz d entsprechende Dosismenge voreingestellt werden kann, die mit der Injektionsvorrichtung abgegeben werden kann. Ebenso können beispielsweise Radialanschläge an der Drehhülse und/oder an den mit der Drehhülse zusammenwirkenden Elementen, also dem Vorschubglied und einem weiteren Element wie z. B. dem Gehäuse vorgesehen sein, welche eine Drehbewegung der Drehhülse begrenzen und z. B. nur eine maximale Drehung von 180° oder beispielsweise zwei volle Umdrehungen ermöglichen. Insbesondere kann mit solchen Anschlägen eine feste Dosis vorgegeben werden, so dass ein Benutzer das Einstellelement z. B. bis zu einem Anschlag eines Elements herauszieht, wobei die voreingestellte Dosis beim Einschieben des Einstellelements abgegeben wird.

Bevorzugt ist an der Drehhülse und/oder an dem mit der Drehhülse verbundenen Einstellelement, wie z. B. einem Bedienknopf, eine Markierung vorgesehen, welche bei einem in die Injektionsvorrichtung eingeschobenen Zustand nicht oder durch z. B. eine Öffnung oder transparentes Material ablesbar ist und welche von einem Benutzer abgelesen oder gesehen werden kann, wenn die Drehhülse und/oder ein mit der Drehhülse verbundenes Bedienelement aus der Injektionsvorrichtung oder dem Gehäuse der Injektionsvorrichtung herausgezogen wurden, so dass es einem Benutzer ermöglicht wird, die aufgrund des Ausschubweges festgelegte eingestellte Dosis abzulesen, die beim Zurückschieben der Drehhülse oder des Bedienknopfes durch die auf die Zahnstange und den Stopfen übertragene Einschubbewegung aus der Injektionsvorrichtung abgegeben wird.

Weiterhin bezieht sich die Erfindung auf eine wie oben beschriebene Injektionsvorrichtung, in welcher eine abzugebende Substanz enthalten ist oder in einer Ampulle eingelegt werden kann und wobei die Dosiervorrichtung mit einem Verdrängungskörper oder Stopfen der Injektionsvorrichtung so gekoppelt werden kann, dass durch die Drehhülse eine abzugebende Dosis eingestellt und ausgeschüttet werden kann.

Die Erfindung wird nachfolgend anhand bevorzugter Ausführungsbeispiele beschrieben. Es zeigen:
Figuren 1A bis 1C eine erfindungsgemäße Injektionsvorrichtung mit einer Dosiervorrichtung in Grundstellung, beim Einstellen und nach dem Ausschütten einer Dosis;
Figuren 2A und 2B die erfindungsgemäße Injektionsvorrichtung mit einer Dosiervorrichtung im Querschnitt; und
Figur 3 die erfindungsgemäße Injektionsvorrichtung mit einer Dosiervorrichtung.

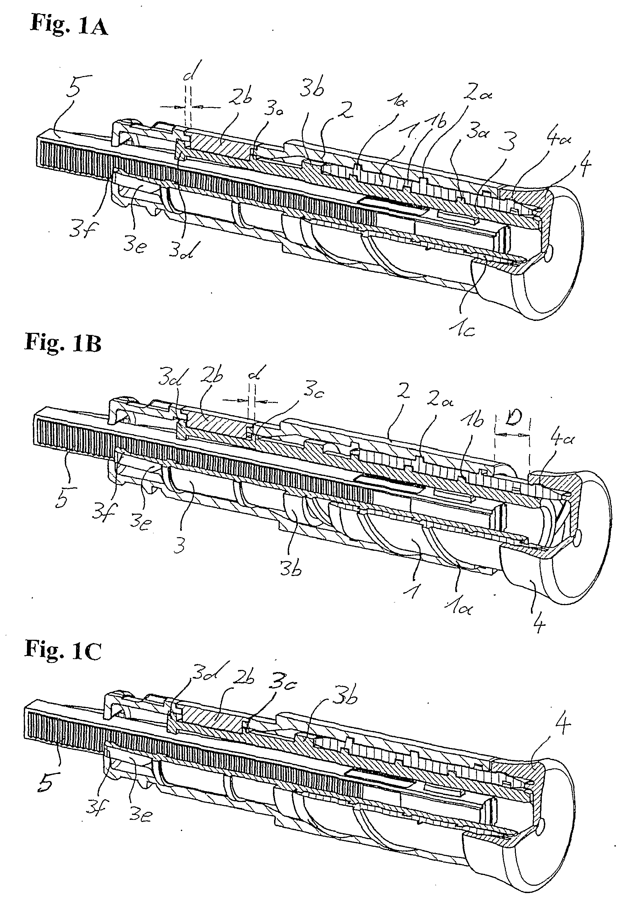

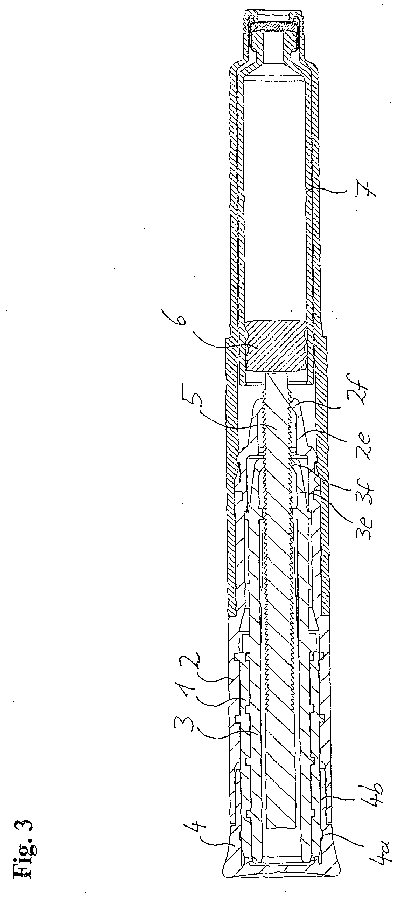

Die Figuren 1A bis 1C zeigen eine erfindungsgemäße Injektionsvorrichtung mit einem Gehäuse 2 und mit einer Dosiervorrichtung, welche ein Innengewinde 2a mit einer ersten Steigung von z. B. 49° hat. Koaxial in dem Gehäuse drehbar gelagert ist eine Drehhülse 1 mit einem Außengewinde 1a mit der gleichen Steigung wie das Innengewinde 2a des Gehäuses 2, welches in das Innengewinde 2a eingreift. Die Drehhülse 1 hat ein Innengewinde 1b mit einer Steigung von z. B. 34°, welche kleiner ist als die Steigung des Außengewindes 1a bzw. des Innengewindes 2a des Gehäuses. Der Unterschied der Gewindesteigungen liegt z. B. im Bereich von 10 bis 15 Grad. Innerhalb der Drehhülse 1 ist verdrehgesichert zum Gehäuse 2 ein Vorschubglied 3 gelagert, welches ein Außengewinde 3a hat, das in das Innengewinde 1b der Drehhülse 1 eingreift. An der in Figur 1A links gezeigten distalen oder Vorderseite des Vorschubgliedes 3 sind einander gegenüberliegend zwei an elastischen Armen 3e befestigte Nasen oder Nocken 3f angebracht, welche in die auf der Außenseite einer Zahnstange 5, welche axial verschiebbar innerhalb des Vorschubgliedes 3 angeordnet ist, vorhandene Zahnung eingreifen können. Die Nasen oder Nocken 3f des Vorschubgliedes 3 sind ebenso wie die Nasen oder Nocken 2f, welche mit dem Gehäuse 2 verbunden und in Figur 3 gezeigt sind, in proximaler Richtung relativ zur Zahnstange bewegbar, wobei die Nasen 2f, 3f entgegen die elastische oder Federkraft der elastischen Arme 2e bzw. 3e nach außen gedrückt werden, um über einen oder mehrere Zähne der Zahnstange 5 hinweg bewegt zu werden. Jedoch werden die Nasen 2f und 3f durch die in Richtung auf die Zahnstange 5 wirkende Vorspannung der elastischen Arme 2e und 3e auf die Zahnstange 5 und so in einen Eingriff mit der Zahnung gedrückt, dass eine Bewegung der Nasen 2f und 3f in distale Richtung der Zahnstange 5 nicht möglich ist bzw. durch den Eingriff der Nasen 2f und 3f in die Zahnung der Zahnstange 5 behindert wird.

Am proximalen Ende ist die Drehhülse 1 mit einem Bedienknopf 4 verbunden und drehbar in diesem durch eine auf der Innenseite des Bedienknopfes 4 vorgesehene umlaufende Nut 4a gelagert, in welche ein auf der Außenseite der Drehhülse 1 vorgesehener umlaufender Ring 1c eingreift. Wird der Bedienknopf 4 von einem Benutzer aus dem Gehäuse 2 herausgezogen, so wird von diesem die Drehhülse 1 mitgenommen, welche aufgrund des in das Innengewinde 2a des Gehäuses 2 eingreifenden Außengewindes 1a relativ zum Gehäuse 2 beim Herausziehen gedreht wird. Diese Drehung der Drehhülse 1 wird aufgrund des sich mit dem Innengewinde 1b der Drehhülse 1 in Eingriff befindlichen Außengewindes 3a des relativ zum Gehäuse 2 verdrehsicher gelagerten Vorschubgliedes 3 in eine Axialbewegung des Vorschubgliedes 3 umgesetzt. Aufgrund der größeren Steigung des Außengewindes 1a der Drehhülse im Vergleich zum Innengewinde 1b der Drehhülse wird ein Ausschubweg D, wie in Figur 1B gezeigt, in einen kleineren Ausschubweg d des Vorschubgliedes 3 untersetzt, so dass kleine Dosismengen präzise eingestellt werden können.

Auf der Außenseite des Vorschubgliedes 3 sind zwei radial nach außen vorstehende axial beabstandete Anschläge 3c und 3d vorgesehen, zwischen welchen das Anschlagselement 2b, welches mit dem Gehäuse 2 verbunden ist, eingreift. In der in Figur 1A gezeigten Ausgangsstellung liegt der distale axiale Anschlag 3c des Vorschubgliedes an der distalen Seite des Anschlagselementes 2b an. Der proximale Anschlag 3d des Vorschubgliedes hat einen Abstand d von der distalen Seite des Anschlagselements 2b.

In Figur 1B ist die Injektionsvorrichtung mit der Dosiervorrichtung nach dem Herausziehen des Bedienknopfes 4 um einen Weg D von z. B. 5 mm gezeigt, was zu der untersetzten Axialbewegung um die Entfernung d von z. B. 0,8119 mm des Vorschubgliedes 3 führt, bis der Anschlag 3d des Vorschubgliedes 3 an der distalen Seite des Anschlagselementes 2b anliegt, wodurch die Ausschubbewegung des Bedienknopfes 4 begrenzt wird. Die Drehhülse 1 wurde dabei z. B. um -90° gedreht.

Beim Herausziehen des Bedienknopfes 4 und der Verschiebung des Vorschubgliedes 3 in proximale Richtung werden die mit dem Vorschubglied 3 verbundenen Nasen oder Nocken 3f in proximale Richtung entlang der durch die mit dem Gehäuse 2 verbundenen Nasen 2f gehaltenen Zahnstange 5 verschoben, so dass die sich einander gegenüberliegenden Nasen 3f z. B. jeweils über einen, zwei oder auch mehr Zähne der Zahnstange 5 nach hinten verschoben werden.

Drückt ein Benutzer auf den Bedienknopf 4 und schiebt diesen wieder zurück in das Gehäuse 2 ein, wie in Figur 1C gezeigt, so wird die Einschubbewegung des Bedienknopfes auf die Drehhülse 1 übertragen, welche sich aufgrund des Gewindeeingriffes mit dem Gehäuse 2 relativ zu diesem um z. B. +90° dreht und durch den Gewindeeingriff mit dem Vorschubglied 3 dieses axial nach vorne schiebt, bis wieder der proximale Anschlag 3c an dem Anschlagselement 2b und die Vorderseite der Drehhülse 1 an einem Anschlag 3b des Vorschubgliedes 3 anliegt. Dabei wird die Zahnstange 5 durch die in die Zahnung eingreifenden Nasen 3f gehalten und zusammen mit dem Vorschubglied 3 in distale Richtung verschoben, wobei die Zahnstange relativ zu dem mit dem Gehäuse 2 verbundenen Nasen 2f um den Weg d verschoben wird, welche nach Beendigung des Vorschubvorganges in Zähne der Zahnstange 5 eingreifen, welche axial in proximale Richtung im Vergleich zur in Figur 1A gezeigten Ausgangsstellung versetzt sind. Diese Vorschubbewegung der Zahnstange 5 wird auf den in Figur 3 gezeigten Kolben 6 übertragen, welcher in die in der Injektionsvorrichtung eingesetzte Ampulle 7 eingeschoben wird und so eine in der Ampulle 7 enthaltene Substanz, wie z. B. Insulin, verdrängt, so dass die der Vorschubbewegung d des Kolbens 6 entsprechende Substanzmenge aus der Ampulle 7 abgegeben wird.

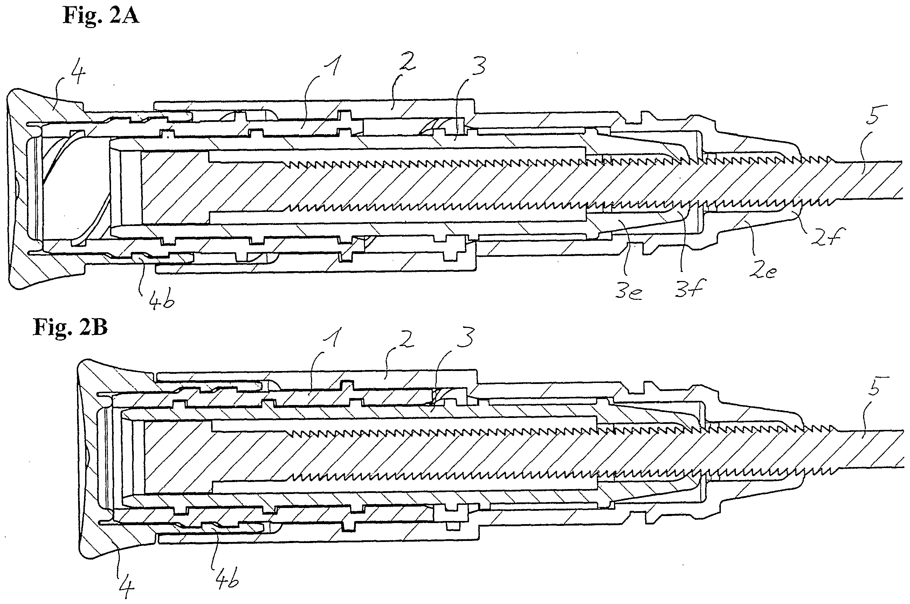

Die Figuren 2A und 2B zeigen im Querschnitt eine weitere Ausführungsform der erfindungsgemäßen Injektionsvorrichtung mit der Dosiervorrichtung, wobei der Bedienknopf 4 axial in das Gehäuse 2 hineinragende Stege 4b oder auch ein zylinderförmig umlaufendes in eine entsprechende Vertiefung des Gehäuses 2 einschiebbares Element 4b aufweist. Beim Herausziehen des Bedienknopfes 4 ist somit nicht die Außenseite der Drehhülse 1 sichtbar, wie z. B. in Figur 1B gezeigt, sondern die Außenseite des Elementes 4b, wie in Figur 2A gezeigt.

Sowohl auf der Außenseite der Drehhülse 1, als auch auf der Außenseite des Elementes 4b können Markierungen angebracht sein, um einem Benutzer Informationen bezüglich einer aufgrund des Ausschubes aus dem Gehäuse 2 eingestellten Dosis z. B. durch farbige Markierungen oder Ringe anzuzeigen.

## Patentansprüche

1. Injektionsvorrichtung mit einer Dosiervorrichtung mit einem Einstellelement (4), einer bevorzugt etwa zylinderförmigen Drehhülse (1), welche mit dem Einstellelement (4) drehbar verbunden ist und ein Außengewinde (1a) und ein Innengewinde (1b) aufweist, welches eine andere Steigung als das Außengewinde (1a) hat, wobei eines der Gewinde (1a, 1b) der Drehhülse (1) mit einem Gewinde (2a) eines Gehäuses der Injektionsvorrichtung im Eingriff steht und das andere der Gewinde (1a, 1b) der Drehhülse (1) mit einem Gewinde (3a) eines Vorschubglieds (3) im Eingriff steht,
**dadurch gekennzeichnet dass** eine Einstellbewegung des Einstellelementes (4) von der Drehhülse (1) durch die beiden Gewindeeingriffe (1a, 2a; 1b, 3a) in eine Dosierbewegung des Vorschubgliedes (3) übersetzt oder untersetzt werden kann.

2. Injektionsvorrichtung nach Anspruch 1, wobei die Steigung desjenigen Gewindes (1b) der Drehhülse (1), welches mit dem Vorschubglied (3) im Eingriff steht, kleiner ist als die Steigung des anderen Gewindes (1a) der Drehhülse (1).

3. Injektionsvorrichtung nach Anspruch 1, wobei die Steigung desjenigen Gewindes (1b) der Drehhülse (1), welches mit dem Vorschubglied (3) im Eingriff steht, größer ist als die Steigung des anderen Gewindes (1a) der Drehhülse (1).

4. Injektionsvorrichtung nach einem der vorhergehenden Ansprüche, wobei der Unterschied der Gewindesteigungen im Bereich von 5 bis 30 oder 10 bis 15 Grad liegt.

5. Injektionsvorrichtung nach einem der vorhergehenden Ansprüche, wobei das Vorschubglied (3) relativ zum Gehäuse (2) verdrehgesichert gelagert ist.

6. Injektionsvorrichtung nach einem der vorhergehenden Ansprüche, wobei an dem Gehäuse (2) ein Innengewinde (2a) vorgesehen ist, in welches das Außengewinde (1a) der Drehhülse (1) eingreift und das Vorschubglied (3) ein Außengewinde (3a) aufweist, das in das Innengewinde (1b) der Drehhülse eingreift.

7. Injektionsvorrichtung nach einem der vorhergehenden Ansprüche, wobei die Gewindesteigungen der Drehhülse (I)5 des Gehäuses (2) und des Vorschubgliedes (3) so gewählt werden, dass die Gewinde nicht selbsthemmend sind.

8. Injektionsvorrichtung nach einem der vorhergehenden Ansprüche, wobei ein Federelement, insbesondere eine Torsionsfeder vorgesehen ist, welche in oder gegen die Drehrichtung der Drehhülse wirkt.

9. Injektionsvorrichtung nach einem der vorhergehenden Ansprüche, wobei das Außengewinde (1a) der Drehhülse (1) koaxial und in radialer Richtung zumindest zum Teil überlappend relativ zu dem Innengewinde (1b) der Drehhülse (1) angeordnet ist.

10. Injektionsvorrichtung nach einem der vorhergehenden Ansprüche, wobei an dem Vorschubglied (3) mindestens eine Rastnase oder Rastnocke (3f) bevorzugt an einem elastischen Element oder Arm (3e) angebracht ist.

11. Injektionsvorrichtung nach einem der vorhergehenden Ansprüche mit einer Zahnstange (5), welche axial verschiebbar innerhalb der Dosiervorrichtung und bevorzugt innerhalb des Vorschubgliedes (3) gelagert ist und mindestens eine oder zwei Zahnreihen an der Außenseite aufweist, in welche mindestens eine elastisch gelagerte Rastnase (3f) des Vorschubgliedes (3) und/oder mindestens eine elastisch gelagerte Rastnase (2f), welche mit dem Gehäuse (2) verbunden ist, eingreifen kann.

12. Injektionsvorrichtung nach einem der vorhergehenden Ansprüche mit mindestens einem radialen und/oder axialen Anschlag (3c, 3d) am Vorschubglied (3) und/oder an der Drehhülse (1) und/oder am Gehäuse (2), um eine Radial- und/oder Axialbewegung des Vorschubgliedes (3) und/oder der Drehhülse (1) relativ zum Gehäuse (2) zu begrenzen.

13. Injektionsvorrichtung nach einem der vorhergehenden Ansprüche, wobei auf einer Außenseite des Einstellelementes (4) und/oder einer Außenseite der Drehhülse (1) Markierungen zur Anzeige einer eingestellten Dosis aufgebracht sind.

14. Injektionsvorrichtung nach einem der vorhergehenden Ansprüche, in welcher eine abzugebende Substanz enthalten ist oder in einer Ampulle (7) eingelegt werden kann.

## Claims

1. Injection device with a dosing device with an adjustment element (4), a preferably approximately cylinder-shaped rotation sleeve (1), which is rotatably connected with the adjustment element (4) and has an external thread (1a) and an internal thread (1b), which has a different pitch than the external thread (1a), wherein one of the threads (1a, 1b) of the rotation sleeve (1) engages a thread (2a) of a housing of the injection device, and the other engages the thread (1a, 1b) of the rotation sleeve (1) with a thread (3a) of the advancing member (3),
**characterised in that** an adjusting movement of the adjusting element (4) can be translated into or fallen short of a dosing movement of the advancing member(3) by the rotation sleeve (1) by means of the two thread engagements (1a, 2a; 1b, 3a).

2. Injection device according to claim 1, wherein the pitch of the respective thread (1b) of the rotation sleeve (1), which engages the advancing member (3), is smaller than the pitch of the other thread (1a) of the rotation sleeve (1).

3. Injection device according to claim 1, wherein the pitch of the respective thread (1b) of the rotation sleeve (1), which engages the advancing member (3), is greater than the pitch of the other thread (1a) of the rotation sleeve (1).

4. Injection device according to one of the preceding claims, wherein the difference in thread pitches lies within a range of 5 to 30 or 10 to 15 degrees.

5. Injection device according to one of the preceding claims, wherein the advancing member (3) is non-rotationally mounted relative to the housing (2).

6. Injection device according to one of the preceding claims, wherein an internal thread (2a) is envisaged on the housing (2), which is engaged by the external thread (1a) of the rotation sleeve (1) and the advancing member (3) has an external thread (3a), which engages the internal thread (1b) of the rotation sleeve.

7. Injection device according to one of the preceding claims, wherein the thread pitches of the rotation sleeve (I)5 of the housing (2) and the advancing member (3) are selected in such a way that the threads are not self-inhibiting.

8. Injection device according to one of the preceding claims, wherein a spring element, in particular a torsion spring, is envisaged, which acts in or against the direction of rotation of the rotation sleeve.

9. Injection device according to one of the preceding claims, wherein the external thread (1a) of the rotation sleeve (1) is arranged coaxially and in a radial direction, at least partly overlapping relative to the internal thread (1b) of the rotation sleeve (1).

10. Injection device according to one of the preceding claims, wherein at least one arresting lug or arresting cam (3f) is affixed to the advancing member (3), preferably on an elastic element or arm (3e).

11. Injection device according to one of the preceding claims, with a gear rod (5), which is mounted axially displaceable within the dosing device and preferably within the advancing member (3), and has at least one or two gear rows on the outside, which can be engaged by at least one elastically mounted arresting lug (3f) of the advancing member (3) and/or at least one elastically mounted arresting lug (2f), which is connected with the housing (2).

12. Injection device according to one of the preceding claims, with at least one radial and/or axial stop (3c, 3d) on the advancing member (3) and/or on the rotation sleeve (1) and/or on the housing (2), for delimiting a radial and/or axial movement of the advancing member (3) and/or the rotation sleeve (1) relative to the housing (2).

13. Injection device according to one of the preceding claims, wherein markings for indicating an adjusted dose are applied on the outside of the adjusting element (4) and/or an outside of the rotation sleeve (1).

14. Injection device according to one of the preceding claims, in which a substance to be dispensed is contained or can be inserted in an ampoule (7).

## Revendications

1. Dispositif d'injection avec un dispositif de dosage avec un élément de réglage (4), un manchon rotatif (1), de préférence de forme approximativement cylindrique, qui est relié de manière rotative avec l'élément de réglage (4) et qui comprend un filetage externe (1a) et un filetage interne (1b), qui présente un pas différent de celui du filetage externe (1a), un des filetages (1a, 1b) du manchon rotatif (1) étant engrené avec un filetage (2a) d'un boîtier du dispositif d'injection et l'autre des filetages (1a, 1b) du manchon rotatif (1) étant emboîté avec un filetage (3a) d'un élément d'avance (3),
**caractérisé en ce qu'**un mouvement de réglage de l'élément de réglage (4) peut être multiplié ou démultiplié par le manchon rotatif (1), à l'aide des deux emboîtements de filetages (1a, 2a ; 1b, 3a), afin d'obtenir un mouvement de dosage de l'élément d'avance (3).

2. Dispositif d'injection selon 1a revendication 1, le pas du filetage (1b) du manchon rotatif (1), qui est emboîté avec l'élément d'avance (3), étant inférieur au pas de l'autre filetage (1a) du manchon rotatif (1).

3. Dispositif d'injection selon la revendication 1, le pas du filetage (1b) du manchon rotatif (1), qui est emboîté avec l'élément d'avance (3), étant supérieur au pas de l'autre filetage (1a) du manchon rotatif (1).

4. Dispositif d'injection selon l'une des revendications précédentes, la différence entre les pas des filetages étant de l'ordre de 5 à 30 ou de 10 à 15 degrés.

5. Dispositif d'injection selon l'une des revendications précédentes, l'élément d'avance (3) étant logé de façon à empêcher une rotation par rapport au boîtier (2).

6. Dispositif d'injection selon l'une des revendications précédentes, un filetage interne (2a) étant prévu sur le boîtier (2), dans lequel le filetage externe (1a) du manchon rotatif (1) s'emboîte et l'élément d'avance (3) comprend un filetage externe (3a) qui s'emboîte dans le filetage interne (1b) du manchon rotatif.

7. Dispositif d'injection selon la revendication 1, les pas des filetages du manchon rotatif (1), du boîtier (2) et de l'élément d'avance (3) étant choisis de façon à ce que les filetages ne soient pas auto-bloquants.

8. Dispositif d'injection selon l'une des revendications précédentes, un élément à ressort, plus particulièrement un ressort de torsion, étant prévu, qui agit dans ou contre le sens de rotation du manchon rotatif.

9. Dispositif d'injection selon l'une des revendications précédentes, le filetage externe (1a) du manchon rotatif (1) étant disposé de manière coaxiale et de manière superposée dans la direction radiale au moins partiellement par rapport au filetage interne (1b) du manchon rotatif (1).

10. Dispositif d'injection selon l'une des revendications précédentes, au moins un embout d'encliquetage ou une came d'encliquetage (3f) étant monté sur l'élément d'avance (3), de préférence au niveau d'un élément ou d'un bras élastique (3e).

11. Dispositif d'injection selon l'une des revendications précédentes, avec une crémaillère (5), qui est logée de manière coulissante axialement à l'intérieur du dispositif de dosage et de préférence à l'intérieur de l'élément d'avance (3) et qui comprend, au moins une ou deux rangées de dents sur le côté extérieur, dans lesquelles au moins un embout d'encliquetage (3f), logé de manière élastique, de l'élément d'avance (3) et/ou au moins un embout d'encliquetage (2f), logé de manière élastique, relié avec le boîtier (2), peut s'emboîter.

12. Dispositif d'injection selon l'une des revendications précédentes, avec au moins une butée radiale et/ou axiale (3c, 3d) sur l'élément d'avance (3) et/ou sur le manchon rotatif (1) et/ou sur le boîtier (2), afin de limiter un mouvement radial et/ou axial de l'élément d'avance (3) et/ou du manchon rotatif (1) par rapport au boîtier (2).

13. Dispositif d'injection selon l'une des revendications précédentes, des marquages pour l'affichage d'une dose réglée étant apposés sur un côté extérieur de l'élément de réglage (4) et/ou sur un côté extérieur du manchon rotatif (1).

14. Dispositif d'injection selon l'une des revendications précédentes, dans lequel une substance à distribuer est contenue ou peut être introduite dans une ampoule (7).
